# DEMANDE DE BREVET EUROPEEN

(11) **EP 1 479 392 A1**
(43) Date de publication de la demande: **24.11.2004**
(21) Numéro de dépôt: 04291099.2
(22) Date de dépôt: 29.04.2004
(51) Int. Cl.: A61K 35/78, A23L 1/30, A23L 1/29

(54) **Utilisation de compléments végétaux d'origine alimentaire, doués de propriétés anti-stress oxydatif dans le cadre de l'effort physique, humain ou animal**

(30) Priorité: 30.04.2003 FR 0305312
(71) Demandeur: Rothfuss, Jacqueline, 67630 Lauterbourg (FR)
(72) Inventeur: Rothfuss, Jacqueline, 67630 Lauterbourg (FR)

(57) **Abrégé**

Utilisation de compléments alimentaires comportant des extraits ou des poudres réalisés à partir des fruits et des légumes, destinés à neutraliser les effets nocifs du stress oxydatif généré par des efforts physiques chez l'homme ou chez l'animal.

## Description

On entend par Stress oxydatif, un état au cours duquel, les oxydants exogènes comme endogènes interviennent lorsque la capacité de défense naturelle de la cellule est dépassée.

La demande de brevet n° 02 11291 concerne l'application cutanée locale d'extraits végétaux alimentaires permettant d'inhiber l'effet nocif des prostanoïdes et de leurs métabolites.

La demande de brevet n° 02 14623 concerne l'utilisation d'extraits végétaux réalisés à partir de fruits et de légumes, caractérisés par leur activité destructrice du peroxyde d'hydrogène, similaire à celle des enzymes : peroxydases et catalases

La présente invention, s'attache au maintien de l'équilibre oxydo-réducteur au cours de l'effort physique chez l'humain ou chez l'animal.

L'exercice physique provoque à un certain niveau d'efforts chez l'homme comme chez l'animal, une augmentation des radicaux libres, conduisant à une production de lipoperoxydes toxiques, générés par une surproduction d'eau oxygénée des macrophages, et par une augmentation de l'activité de la xanthine-oxydase. Concernant la production de xanthine-oxydase, on a pu constater chez le rat nageant jusqu'à épuisement, un accroissement de cette enzyme de l'ordre de 95% par rapport aux animaux témoins, avec augmentation significative des lipoperoxydes pulmonaires (REDDY et Col-Biochemistry Internat. 1992 26 (5) 863 ) Cette production d'oxygène actif est capable de causer des dommages aux protéines et aux acides nucléiques.

La lipoperoxydation conduit à une oxydation du glutathion réduit, principal protecteur antioxydant de l'organisme humain et animal, à l'altération des érythrocytes, et à une augmentation significative du malondialdéhyde par exemple dans la sueur des sportifs (KANTER et Col-Annals of sports Med. 1986 3 39), (WILBUR et Col. Arch. of biochem. 1949 24 305) ainsi que dans le plasma du cheval ( FYCI et Col-Proc.Natl. Acad . Sci. USA 1980 80 1521.)

Chez les coureurs de fond ou les chevaux de course subissant un stress d'endurance, il a été constaté la formation de lésions oxydatives au niveau des muscles ( FUNES J- KAREL M-US Army Res. Office 1984 25 juin.)

Il est évident qu'après une course ou un effort physique intense, que ce soit chez l'humain ou l'animal, nécessitant une consommation importante d'oxygène, il y ait une augmentation sensible du processus oxydatif se traduisant par une production accrue d'eau oxygénée due au stress.

Les dommages organiques provoqués par le stress oxydatif sont donc liés aux niveaux des systèmes antioxydants enzymatiques et non enzymatiques, conduisant à une réduction de la vie humaine ou animale.

Lors d'un effort physique intense, il semble impératif de fournir à l'homme comme à l'animal les moyens de neutraliser l'action agressive du stress oxydatif par le choix judicieux de compléments alimentaires à hautes capacités anti-lipoperoxydes, anti-peroxyde d'hydrogène et possédant de plus la propriété d'inhiber la xanthine -oxydase.

La présente invention a pour objet d'utiliser des compléments alimentaires, doués de propriétés permettant de neutraliser les effets nocifs du stress oxydatif générés par des efforts physiques chez l'homme ou l'animal, pouvant avoir une conséquence sur leur état de santé et leur longévité.

Ces compléments alimentaires seront réalisés selon l'invention, en sélectionnant les légumes ou les fruits en fonction de leur triple activité antioxygène actif. Ils seront utilisés pour l'homme et pour l'animal, soit sous forme d'extraits solides ou aqueux, soit directement par déshydratation du végétal sous forme pulvérulente.

On utilisera préférentiellement les végétaux alimentaires dont les capacités antioxygène actif seront les plus élevées. A titre d'exemples non limitatifs, le tableau ci-dessous donne quelques résultats de mesures permettant d'effectuer une sélection ( nécessaire, étant donné la variabilité des teneurs des végétaux en substances antioxygènes, liée à de multiples paramètres).

C'est ainsi, qu'il a été découvert chez l'ortie une forte triple action par rapport à d'autres végétaux alimentaires.

| **VEGETAUX** ( pour 1g) | **Oxygène Lipoperoxydique Eliminé (en µg/g)** | **Oxygène du Peroxyde d'hydrogène éliminé** | **Inhibition de la Xanthine- oxydase** |
|---|---|---|---|
| **Feuilles** | | | |
| Ortie n°1 | 3200 | 1480 | + |
| Ortie n°2 | 3660 | 1720 | + |
| Betterave | 1600 | 1120 | + |
| Carotte | 1200 | 960 | + |
| Poivron vert | 1050 | 840 | + |

Tous les autres légumes et fruits accusent des chiffres très inférieurs à ceux de l'ortie.

| **Extraits** | | | |
|---|---|---|---|
| Ortie n° 1 | 3920 | 2336 | + |
| Ortie n°2 | 3760 | 2880 | + |
| Poivron rouge | 1600 | 700 | + |
| Chou brocoli | 1200 | 960 | + |
| Epinard | 1100 | 960 | + |

En fonction des mesures effectuées sur un nombre important de fruits et de légumes, afin de neutraliser les conséquences de l'agressivité du stress oxydatif, on constate que l'ortie est le végétal ayant les capacités antioxygènes les plus élevées.

A titre d'exemples non limitatifs, les compléments alimentaires obtenus à partir de végétaux alimentaires seront utilisés en fonction de leur triple capacité antioxygène, déterminée par mesures. Ils pourront être employés sous forme solide ou liquide (comprimés, poudres, gélules, solutions, ampoules) Pour ce qui concerne le cas particulier des chevaux de course, à qui on demande d'intenses efforts, on aura recours soit à la forme poudre, soit à la forme liquide.

Quant à la protection métabolique générale des sportifs soumis à des efforts intenses, celle-ci sera assurée par des extraits utilisés sous forme de comprimés ou de gélules, permettant l'emploi de doses protectrices importantes.

La présente invention est caractérisée par la détermination de trois indices fondamentaux :
- Anti-peroxyde d'hydrogène
- Anti-lipoperoxydes
- Par contrôle de l'inhibition de la xanthine-oxydase

A partir des chiffres du tableau, et par rapport aux quantités de poudres ou d'extraits utilisés, on déterminera l'indice de protection anti-oxydatif, exprimé par deux chiffres, l'un se rapportant aux lipoperoxydes, l'autre au peroxyde d'hydrogène.

### Exemples de compositions anti-stress oxydatif :

### Pour les Sportifs

| | |
|---|---|
| *Comprimés 500mg* **Extrait d'ortie** | 450mg |
| Agglomérant Indice de protection (1760/1801) | 50mg |
| **Extrait de poivron rouge** | 450mg |
| Agglomérant Indice de protection (720/315) | 50mg |

### Pour les chevaux de course

| | |
|---|---|
| **Poudre d'ortie** Indice de protection (32000/14800) | 10g |
| **Feuilles de betterave** (poudre) Indice de protection (32000/22400) | 20g |
| **Extrait d'ortie** | 10g |
| Eau Indice de protection (39200/23360) | 80ml |

## Revendications

1. Utilisation de compléments alimentaires doués de propriétés permettant de lutter contre le stress oxydatif, généré par des effort physiques chez l'homme ou l'animal, **caractérisés en ce qu'**ils comportent des végétaux alimentaires soit sous forme d'extraits solides ou aqueux, soit sous forme pulvérulente par déshydratation du végétal, sélectionnés en fonction de leur triple capacité anti-oxygènes actifs.

2. Utilisation de compléments alimentaires selon la revendication 1, **caractérisés en ce qu'**ils sont doués d'une triple capacité anti-oxygènes actifs déterminée par mesures : Anti-lipoperoxydes, anti-peroxyde d'hydrogène et anti-xanthine-oxydase.

3. Utilisation de compléments alimentaires selon les revendications 1 et 2, **caractérisés en ce qu'**ils sont employés pour l'homme ou l'animal sous forme solide ou liquide ( comprimés, poudres, gélules, solutions aqueuses, ampoules)
